## Europäisches Patentamt
## European Patent Office
## Office européen des brevets

(19)

(11) Veröffentlichungsnummer: **0 147 588**
**B1**

(12) **EUROPÄISCHE PATENTSCHRIFT**

(45) Veröffentlichungstag der Patentschrift:
18.03.87

(21) Anmeldenummer: 84113506.0

(22) Anmeldetag: 06.11.84

(51) Int. Cl.⁴: **C 09 J 7/02**, C 09 J 3/16,
A 61 L 15/06

(54) Selbsthaftende Flächengebilde, Verfahren zu deren Herstellung und deren Verwendung.

(30) Priorität: 17.11.83 DE 3341555

(43) Veröffentlichungstag der Anmeldung:
10.07.85 Patentblatt 85/28

(45) Bekanntmachung des Hinweises auf die Patenterteilung:
18.03.87 Patentblatt 87/12

(84) Benannte Vertragsstaaten:
AT BE CH DE FR GB IT LI NL SE

(56) Entgegenhaltungen:
EP-A-0 057 839
FR-A-2 128 408
FR-A-2 226 453
FR-A-2 237 946

(73) Patentinhaber: BAYER AG, Konzernverwaltung RP Patentabteilung, D-5090 Leverkusen 1 Bayerwerk (DE)

(72) Erfinder: von Bittera, Miklos, Max- Scheler-Strasse 7, D-5090 Leverkusen 3 (DE)
Erfinder: Schäpel, Dietmar, Dr., Johanniterstrasse 15, D-5000 Koeln 80 (DE)
Erfinder: von Gizycki, Ulrich, Dr., Wiembachallee 24, D-5090 Leverkusen 3 (DE)
Erfinder: Rupp, Roland, Dr., Solinger Strasse 18, D-5653 Leichlingen 2 (DE)

EP 0 147 588 B1

**Beschreibung**

Die vorliegende Erfindung betrifft neuartige selbsthaftende Flächengebilde, bestehend aus mindestens einer Trägerschicht und mindestens einer Haftschicht auf Basis von Polyurethan-Gel. Die Flächengebilde können durch Beschichtung von verschiedenartigsten Trägermaterialien mit einem Reaktionsgemisch, das aus Di- und/oder Polyisocyanaten und einer überschüssigen Menge an höhermolekularen Polyolen besteht, und dessen nachfolgende Härtung erhalten werden und z.B. als hautverträgliches Heftpflaster für medizinische Zwecke oder als Haftkleber für technische Einsätze Verwendung finden.

Selbstklebende Flächengebilde, wie z.B. medizinische Pflaster, sind in vielfältigen Ausführungsformen bekannt. Die Hautpflaster für medizinische Zwecke weisen üblicherweise klebende Flächen auf Basis von Kautschuk-Harzen oder von Polyacrylaten auf. Die Pflaster mit Kautschuk-Klebeflächen haben den Nachteil, daß schwierig zu entfernende Reste auf der Haut zurückbleiben, wenn diese Pflaster wieder abgenommen werden. Außerdem führen diese Pflaster oftmals zu Hautreizungen. Die Nachteile von Pflastern mit Haftflächen auf Polyacrylat-Basis liegen in den teilweise auftretenden Hautrötungen bis zu Hautreizungen und in den Hauterweichungsprozessen, die sich allgemein bei ungenügendem Luftzutritt zeigen. Diese Nachteile treten insbesondere dann verstärkt auf, wenn Polyacrylat-Haftflächen sich länger als einen Tag auf der Haut befinden und auch dann, wenn die durch den Haftkleber bedeckte Fläche ein größeres Ausmaß hat.

Für technische Zwecke verwendete selbsthaftende Flächengebilde, deren haftende Wirkung auf Polyvinylchlorid (PVC) basiert (wobei PVC als haftende Beschichtung eines Trägermaterials oder auch als selbsthaftende Folie verwendet wird), weisen den Nachteil auf, daß eine ausreichende Haftwirkung nur auf glatten, ebenen Untergründen erreicht wird. Darüber hinaus wirken die im PVC allgemein enthaltenen niedermolekularen Weichmacher aufgrund ihrer chemischen Struktur teilweise wie Lösungsmittel und/oder vermögen in die Untergründe zu migrieren.

Es wurde nun gefunden, daß man selbsthaftende Flächengebilde erhält, die auch auf rauhen und unebenen Oberflächen gut haften, praktisch rückstandsfrei wieder entfernbar und zusätzlich hautverträglich sind, wenn man beliebige Trägermaterialien mit einem Polyurethan-Reaktionsgemisch beschichtet, das unter Bildung eines strukturfesten, hochelastischen Gels aushärtet.

Gegenstand der Erfindung sind somit selbsthaftende Flächengebilde, enthaltend mindestens eine Trägerschicht und mindestens eine Haftschicht, wobei eine der beiden Oberflächen des Flächengebildes mindestens teilweise mit einer Haftschicht bedeckt ist, dadurch gekennzeichnet, daß diese Haftschicht ein Gel ist, welches

(A) 15-62 Gew.-%, vorzugsweise 20-57 Gew.-%, besonders bevorzugt 25-47 Gew.-%, bezogen auf die Summe aus (A) und (B), eines kovalent vernetzten Polyurethans als hochmolekulare Matrix und

(B) 85-38 Gew.-%, vorzugsweise 80-43 Gew.-%, besonders bevorzugt 75-53 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0 - 100 Gew.-%, bezogen auf die Summe aus (A) und (B), an Füll- und/oder Zusatzstoffen

enthält, und welches erhältlich ist durch Umsetzung einer Mischung von

a) einem oder mehreren Polyisocyanaten,

b) einer oder mehreren Polyhydroxylverbindungen mit einem mittleren Molekulargewicht zwischen 1000 und 12 000, und einer mittleren OH-Zahl zwischen 20 und 112,

c) gegebenenfalls Katalysatoren für die Reaktion zwischen Isocyanat- und Hydroxylgruppen sowie gegebenenfalls

d) aus der Polyurethanchemie an sich bekannten Füll- und Zusatzstoffen,

wobei diese Mischung im wesentlichen frei von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3 und 6 beträgt und die Isocyanatkennzahl (K) der Formel

$$K = \frac{100 \pm X}{(F_I \cdot F_p) - 1} + 7$$

gehorcht, in welcher $X \leqslant 120$, vorzugsweise $X \leqslant 100$, besonders bevorzugt $X \leqslant 90$ ist und die Kennzahl K bei Werten zwischen 15 und 70 liegt.

Die angegebenen Mittelwerte von Molekulargewicht und OH-Zahl sind als Zahlenmittel zu verstehen.

Gegenstand der Erfindung ist ferner ein Verfahren zur Herstellung selbsthaftender Flächengebilde auf Basis von mit Polyurethan-Gel beschichteten Trägermaterialien; das Verfahren ist dadurch gekennzeichnet, daß man die oben definierten zur Gelbildung befähigten Reaktionsgemische auf die Oberfläche eines Trägermaterials

2

nach Direkt- oder Umkehrverfahren durch Gießen, Rakeln oder Sprühen aufbringt, wobei die Oberfläche mit dem gelbildenden Reaktionsgemisch gegebenenfalls nur teilweise bedeckt wird.

Gegenstand der Erfindung ist schließlich auch die Verwendung der selbsthaftenden Flächengebilde in der Medizin, insbesondere als Heft- oder Wundpflaster, Bandage oder Mullbinde.

Die erfindungsgemäßen Flächengebilde können aus den an sich aus der Polyurethanchemie bekannten Ausgangsverbindungen nach an sich bekannten Verfahren hergestellt werden, wie sie z.B. in DE-OS 3 103 499 und DE-OS 3 103 500 beschrieben werden. Wesentlich ist jedoch, daß bei der Auswahl der gelbildenden Komponenten die oben definierten Bedingungen eingehalten werden, da sonst anstelle von selbsthaftenden Gelschichten klebfreie, elastische Gele erhalten werden.

Erfindungsgemäß bevorzugte Polyhydroxylverbindungen sind Polyetherpolyole, wie sie in den oben genannten Deutschen Offenlegungsschriften ausführlich genannt sind. Als Polyisocyanatkomponente sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatische Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen. Die TDI-Typen können auf Grund von Modifizierungen, wie Biuretisierung oder Trimerisierung, auch höherfunktionelle Anteile beinhalten.

Erfindungsgemäß werden die Ausgangskomponenten so gewählt, daß im gelbildenden Reaktionsgemisch die mittlere NCO-Funktionalität zwischen 2 und 4, die mittlere Polyol-Funktionalität zwischen 3 und 6 und die Isocyanatkennzahl zwischen 15 und 70, vorzugsweise zwischen 18 und 55, besonders bevorzugt zwischen 20 und 45, liegen.

Die aus Polyurethan-Gel bestehenden Haftschichten der erfindungsgemäßen Flächengebilde können gegebenenfalls aus der Polyurethan-Chemie an sich bekannte Zusatzstoffe enthalten, wie z.B. Füllstoffe und Kurzfasern auf anorganischer oder organischer Basis, Metallpigmente, wasserbindende Mittel, oberflächenaktive Substanzen oder flüssige Streckmittel wie Substanzen mit einem Siedepunkt von über 150° C.

Als anorganische Füllstoffe seien beispielsweise Schwerspat, Kreide, Gips, Kieserit, Soda, Titandioxid, Ceroxid, Quarzsand, Kaolin, Ruß und Mikrohohlkugeln genannt.

An organischen Füllstoffen können z.B. Pulver auf Basis von Polystyrol, Polyvinylchlorid, Harnstoff-Formaldehyd und Polyhydrazodicarbonamid (z.B. aus Hydrazin und Toluylendiisocyanat) eingesetzt werden.

Als Kurzfasern kommen z.B. Glasfasern von 0,1 - 1 mm Länge oder Fasern organischer Herkunft, wie z.B. Polyester- oder Polyamidfasern, in Frage. Metallpulver, wie z.B. Eisen- oder Kupferpulver, können ebenfalls bei der Gelbildung mitverwendet werden. Um den erfindungsgemäßen Gelen die gewünschte Färbung zu verleihen, können die bei der Einfärbung von Polyurethanen an sich bekannten Farbstoffe oder Farbpigmente auf organischer oder anorganischer Basis verwendet werden, wie z.B. Eisenoxid- oder Chromoxidpigmente, Pigmente auf Phthalocyanin- oder Monoazo-Basis. Das bevorzugte wasserbindende Mittel sind Zeolithe. Als oberflächenaktive Substanzen seien z.B. Cellulosepulver, Aktivkohle, Kieselsäurepräparate und Chrysotil-Asbest genannt.

Als flüssige Streckmittel können beispielsweise alkyl-, alkoxy- oder halogen-substituierte aromatische Verbindungen, wie Dodecylbenzol, m-Dipropoxybenzol oder o-Dichlorbenzol, halogenierte aliphatische Verbindungen, wie chlorierte Paraffine, organische Carbonate, wie Propylencarbonat, Carbonsäureester, wie Dioctylphthalat, Ethylstearat, Laurinsäurehexylester, Isopropylmyristat, Isopropylpalmitat oder Dodecylsulfonsäureester oder organische Phosphorverbindungen, wie Tricresylphosphat, verwendet werden.

Weiterhin können als flüssige Streckmittel auch höhermolekulare Polyole eingesetzt werden deren Hydroxylgruppen verethert, verestert oder urethanisiert sind, sowie Paraffinöle und Silikonöle.

Der Gehalt an Füllstoffen und Streckmitteln in der Gelschicht kann bis zu 50 Gew.-%, bezogen auf das Gesamtgewicht des Gels, betragen.

Zur Modifizierung der Hafteigenschaften der Gelschicht können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Die Schichtdicke des Gels kann z. B. zwischen 0,001 mm und 5 mm, vorzugsweise zwischen 0,01 mm und 2 mm, besonders bevorzugt zwischen 0,1 mm und 1 mm, liegen.

Die in den erfindungsgemäßen selbsthaftenden Flächengebilden enthaltenen Trägermaterialien können unterschiedlichster Herkunft sein, d.h. Materialien auf Basis von natürlichen, halb- oder vollsynthetischen Rohstoffen sowie organischen oder anorganischen Ursprungs sind verwendbar. Beispielsweise lassen sich Kunststoff- und Metallfolien, Matten, Vliese, Gewirke, Gestricke oder Gewebe aus organischem oder anorganischem Fasermaterial, Papier, Pappe, Holz, Leder und Schaumstoff-Folien oder auch Kombinationen aus diesen Trägermaterialien einsetzen. Für die medizinische Anwendung bevorzugt sind luft- und feuchtigkeitsdurchlässige Flächengebilde, z.B. mikro- und makroporöse Kunststofolien, elastische textile Trägermaterialien, insbesondere Stretchgewebe, und Mullbinden.

Die Herstellung der in den erfindungsgemäßen Flächengebilden enthaltenen Gele kann auf verschiedene Weise erfolgen.

Man kann z.B. nach dem one-shot- oder dem Prepolymer-Verfahren arbeiten. Beim one-shot-Verfahren werden alle Komponenten, d.h. Polyole, Di- und/oder Polyisocyanate, die Isocyanat-Polyadditionsreaktion beschleunigende Katalysatoren und gegebenenfalls Füll- und Zusatzstoffe auf einmal zusammengegeben und intensiv miteinander vermischt.

Beim Prepolymer-Verfahren sind zwei Arbeitsweisen möglich. Entweder stellt man zunächst ein Isocyanat-Prepolymer her, indem man einen entsprechenden Anteil der Polyolmenge mit der gesamten, für die Gelbildung vorgesehenen Isocyanatmenge umsetzt, und fügt dann dem erhaltenen Prepolymer die restliche Menge an Polyol sowie gegebenenfalls Füll- und Zusatzstoffen zu und mischt intensiv. Oder man setzt die gesamte, für die Gelbildung vorgesehene Menge an Polyol mit einem Teil der Isocyanatmenge zu einem Hydroxyl-Prepolymer um und mischt anschließend die restliche Menge an Isocyanat zu.

Eine erfindungsgemäß besonders vorteilhafte Arbeitsweise ist eine Variante aus dem one-shot-Verfahren und dem Hydroxyl-Prepolymer-Verfahren. Hierbei werden das Polyol bzw. Polyolgemisch, gegebenenfalls die Füll- und Zusatzstoffe, der Katalysator und zwei verschiedene Diisocyanate in einem Schuß zusammengegeben und intensiv vermischt, wobei ein Diisocyanat aromatischer und ein Diisocyanat aliphatischer Natur ist. Man kann davon ausgehen, daß durch die stark unterschiedliche Reaktivität der beiden Diisocyanate zunächst ein Hydroxylprepolymer entsteht, das sodann innerhalb von Minuten mit dem anderen Diisocyanat unter Gelbildung reagiert.

Bei diesen Verfahrensweisen kann die Förderung, Dosierung und Mischung der Einzelkomponenten oder Komponentengemische mit den für den Fachmann in der Polyurethan-Chemie an sich bekannten Vorrichtungen erfolgen.

Die Herstellung der erfindungsgemäßen Flächengebilde kann kontinuierlich oder diskontinuierlich erfolgen. Die Arbeitsweise hängt von den vorgegebenen, mit einer Haftschicht zu versehenden Flächengebilden ab. Wenn man bereits zugeschnittene Trägermaterialien vorliegen hat, ist oftmals eine diskontinuierliche Arbeitsweise vorteilhaft. Die kontinuierliche Arbeitsweise empfiehlt sich bei der Beschichtung von Trägermaterialien, die in endloser Form, z.B. als Rollenware, vorliegen. Der Auftrag der gelartigen Haftschicht auf das Trägermaterial kann dabei direkt oder nach dem Umkehrverfahren erfolgen. Das gelfähige Reaktionsgemisch läßt sich bei den genannten Verfahren auch, bevor es durch die Reaktion erstarrt, rakeln oder Versprühen. Das Sprühverfahren ermöglicht z.B. die einfache, punktuelle Beschichtung von grobmaschigen Geweben.

Eine vorteilhafte Nachbehandlung der erfindungsgemäßen Flächengebilde ist die Bestrahlung mit gamma-Strahlen, wodurch die Haftfähigkeit der Gelschicht verbessert wird, d.h., die Haftfestigkeit der Flächengebilde gegenüber beliebigen Substraten wird erhöht.

Ein wesentlicher Vorteil der erfindungsgemäßen Flächengebilde beim Anwendungsfall als medizinisches Pflaster ist die gute Hautverträglichkeit. Es zeigen sich keine Mazerationen oder sonstige durch Luft- und Wasserdampfundurchlässigkeit hervorgerufene Hautschäden. Außerdem lassen sich die erfindungsgemäßen Flächengebilde als Pflaster praktisch rückstandsfrei und mit leichtem Zug wieder von der Haut ablösen, ohne daß Epilation auftritt.

Eine besondere Ausführungsform der erfindungsgemäßen Flächengebilde stellen somit Wundverbände dar, die aus einem eine selbsthaftende Polyurethan-Gelschicht aufweisenden Trägermaterial bestehen, auf dem ein Wundkissen zentral angeordnet ist, um die Wunde nach den Seiten hin rundum abzuschließen. Hierbei kann das Trägermaterial z. B. aus einer Kunststoffolie auf Basis von z. B. Polyurethan (PUR) oder PVC, aus einer Schaumfolie auf Basis von z. B. PVC, PUR oder Polyethylen oder vorzugsweise textilen Faservliesen auf Basis von z. B. Viskosefasern sowie Geweben auf Basis von z. B. Kunstseide oder Stretchgewirken auf Basis von z. B. PUR/Polyamid-Garngemischen bestehen. Als saugfähiges zentrales Wundkissen können die als wundverträglich bekannten Materialien wie z. B. Baumwollmull, PUR-Schaumstoffolie oder textile Viskose-Vliesstoffe mit Gewebestruktur verwendet werden. Bevorzugt sind aber Kombinationen aus einer Wundauflage auf Basis von z. B. Baumwollmull oder textilem Viskose-Vliesstoff, der gegebenenfalls aluminiumbedampft ist, und einer Saugschicht auf Basis von z. B. Zellstoff, Viskose oder Viskose/Baumwolle (25:75)-Gemischen. Das Wundkissen kann auch aus einem Wundsekrete aufnehmenden chemisch vernetzten Gel, z. B. einem PUR-Gel gemäß EP-A 0 057 839 bestehen, wobei zur Verstärkung der Saugfähigkeit das Gel gegebenenfalls aufgeschäumt ist, also ein Schaumgel darstellt. Derartige Wundverbände können im Bereich der Wundversorgung bei trockenen und leicht sezernierenden Wunden, in spezieller Ausführungsform, d. h. mit einem weichen zentralen Polsterkissen, auch als Augenverband oder Hühneraugenverband Anwendung finden.

Als eine für die Versorgung kleiner Wunden geeignete Variante des Wundverbandes sind Wundschnellverbände herstellbar, die z. B. aus den gleichen Materialkombinationen wie die Wundverbände bestehen können, aber anstelle des zentralen Wundkissens ein durchlaufendes Wundkissen aufweisen.

Eine weitere Ausführungsform der erfindungsgemäßen Flächengebilde stellen starre (nicht dehnbare) und elastische Binden auf Basis von Baumwolle oder Baumwolle/PUR- bzw. Baumwolle/Polyamid-Garngemischen dar, wobei die Binden durch Benetzung oder Beschichtung mit dem selbsthaftenden PUR-Gel nichtrutschend ausgerüstet sind. Derartige Binden können für Kompressions- und Stützverbände sowie als Sportbandagen, vorteilhafterweise an Gelenken oder konischen Körperteilen, verwendet werden. Die bei elastischen Binden jeweils gewünschte Dehnbarkeit wird üblicherweise neben der Garnart insbesondere durch die Technik des Webens, Wirkens oder Strickens der Binden, die sodann mit Gel versehen werden, erzielt.

Die im medizinischen Bereich für unterschiedliche Zwecke zur Anwendung gelangenden Fixierstreifen bestehen erfindungsgemäß aus einem Trägermaterial, das mit einer Polyurethan-Gelhaftschicht versehen ist. Als Trägermaterial können hierbei z. B. Kunststoffolien auf Basis von z. B. PVC oder PUR, Schaumfolien auf Basis von z. B. Polyethylen, PVC oder PUR, textile Faservliese auf Basis von z. B. Viskose, Gewebe auf Basis von z. B. Kunstseide, Baumwolle oder bevorzugt Stretchgewirke auf Basis von z. B. PUR/Polyamid-Garngemischen zum Einsatz gelangen. Durch Stanzung von geeigneten Lochreihen oder gezackten Kanten lassen sich die Fixierstreifen in von Hand reißbarer Form ausführen. Die erfindungsgemäßen Fixierstreifen können verwendet werden, um z. B. Sonden, Katheter, Meßelemente, Verbände, Salbenkompressen, Wundkompressen, Augenkompressen oder Nabelkompressen zu befestigen.

Eine für die transdermale Wirkstoffverabreichung geeignete Ausführungsform eines wirkstoffhaltigen Hautpflasters besteht erfindungsgemäß aus einem mit einer Polyurethan-Gelhaftschicht versehenen Trägermaterial, auf dem ein wirkstoffhaltiges Kissen zentral angeordnet ist. Dabei befindet sich zwischen gelbeschichtetem Trägermaterial und wirkstoffhaltigem Kissen eine für den jeweiligen Wirkstoff diffusionsdichte Trennschicht aus beispielsweise Aluminiumfolie, die zur Seite des Wirkstoffkissens hin gegebenenfalls mit einem siegelfähigen Film, z. B. auf Basis von EVA, versehen ist, um mittels einer siegelfähigen Deckschicht den Wirkstoff dicht abschließen zu können. Als Trägermaterial für das wirkstoffhaltige Kissen können in Abhängigkeit von Anwendungszweck und Wirkstoffart gelartige Massen auf Basis von z. B. Polyurethan, PVC oder Polyisobutylen sowie Vliese auf Basis von z. B. Viskose zur Anwendung gelangen. Das die Gelhaftschicht aufweisende Trägermaterial des Wirkstoffpflasters kann aus den gleichen Materialien bestehen, wie sie für den Fall der Wundverbände oben beschrieben sind.

Die verschiedenen Pflaster, Binden und Verbände können mit dem die Haftwirkung ergebenden Gel durchgängig oder auch nur teilweise beschichtet sein; sie können mikro- oder grobporös oder auch gelocht vorliegen. So ist es z. B. möglich, Mullbinden oder andere nichtelastische oder elastische Binden nur in kleinen Bereichen (z. B. an den Längsseiten, stegförmig quer zur Wickelrichtung oder punktuell) mit der selbsthaftenden Gelmasse zu versehen, z. B. zur Erzielung eines nicht rutschenden Effektes.

Ein weiterer Vorteil der erfindungsgemäßen Flächengebilde ist deren Fähigkeit, auch auf rauhen, unebenen und stark konturierten Untergründen zu haften. Daher können die erfindungsgemäßen Flächengebilde in vielfältigen Ausführungsformen im technischen Bereich Verwendung finden, z.B. als Haftfläche bei Merk- und Informationstafeln; als Material zur vorübergehenden Reparatur zerrissener Stoffe oder gebrochener Teile; als Befestigungshilfsmittel für Werbematerial; in Form von zerschneidbaren Haftfolien oder -geweben als Gestaltungsmaterial für Kinder; als Haftfläche für Insekten; als Haftetiketten; als Klebefläche bei Briefumschlägen; als Schutzfolie auf Autofenstern gegen nächtliches Beschlagen mit Eisschichten.

Die folgenden Beispiele erläutern die vorliegende Erfindung.

Mengenangaben sind als Gewichtsprozente bzw. Gewichtsteile zu verstehen, sofern nichts anderes angegeben ist.

In den Beispielen wurden die folgenden Polyisocyanate bzw. Polyole eingesetzt:

Polyisocyanat 1:

1,6-Hexamethylendiisocyanat

Polyisocyanat 2:

Handelsübliches biuretisiertes 1,6-Hexamethylen-diisocyanat mit einer mittleren NCO-Funktionalität von 3,6, einem NCO-Gehalt von 21 % und einem mittleren Molekulargewicht (Zahlenmittel) von ca. 700 (Desmodur[z] N der Bayer AG).

Polyisocyanat 3:

Isomerengemisch aus 80 % 2,4- und 20 % 2,6-Toluylendiisocyanat.

Polyisocyanat 4:

Durch Präpolymerisierung mit Tripropylenglykol verflüssigtes 4,4'-Diisocyanatodiphenylmethan; mittlere NCO-Funktionalität: 2,05; NCO-Gehalt: 23 %.

Polyisocyanat 5:

Durch Trimerisierung modifiziertes 1,6-Hexamethylendiisocyanat, mittlere NCO-Funktionalität : 3,4; NCO-Gehalt : 21,5 %, mittleres Molekulargewicht (Zahlenmittel) = ca. 675.

Die in den Beispielen verwendeten Polyether-Polyole sind in der nachfolgenden Tabelle zusammengestellt.

Sie wurden durch Anlagerung von Propylenoxid und gegebenenfalls Ethylenoxid an die angegebenen Startermoleküle in an sich bekannter Weise hergestellt.

| Polyol Nr. | Propylenoxid % | Ethylenoxid % | Starter-molekül | OH-Zahl | OH-Funktio-nalität |
|---|---|---|---|---|---|
| 1 | 80 | 20 | PET | 36 | 4 |
| 2 | 100 | - | Sorbit | 46 | 6 |
| 3 | 73 | 27 | Sorbit | 30 | 6 |
| 4 | 45 | 55 | TMP | 56 | 3 |
| 5 | 100 | - | PET | 72 | 4 |
| 6 | 100 | - | TMP | 56 | 3 |
| 7 | 90 | 10 | Sorbit | 83 | 6 |
| 8 | 100 | - | EDA | 61 | 4 |
| 9 | 83 | 17 | TMP | 35 | 3 |
| 10 | 100 | - | PET | 45 | 4 |

PET = Pentaerythrit

TMP = Trimethylolpropan

EDA = Ethylendiamin

**Beispiel 1**

100 Teile Polyether 1 und 6,1 Teile Polyisocyanat 4, sowie 0,6 Teile Dibutylzinndilaurat werden innerhalb von einer Minute intensiv vermischt und auf Siliconpapier zu einer 0,5 mm starken Schicht ausgegossen. Nach 10 Minuten bbeginnt die Gelbildungsreaktion unter langsamer Viskositätserhöhung der Reaktionsmischung. Es wird ein elastisches Gewirke auf Basis von Polyamid/Polyurethan-Fasern faltenfrei auf den hochviskosen Film der Reaktionsmischung aufgelegt. Nach 20 Minuten ist das Gemisch zum Gel erstarrt. Der gelbeschichtete Stretchstoff wird in Stücke von jeweils 12 x 10 cm Größe zerteilt und auf der gelbeschichteten Seite mit jeweils 4 x 6 cm großen Wundmull-Stücken in der Weise versehen, daß rundum am Rand eine jeweils 3 cm breite haftfähige Gelschicht erhalten bleibt. Ein derartiger Wundverband ist für die Versorgung von Wunden an stark der Bewegung unterliegenden Körperstellen, wie z. B. Gelenken, geeignet.

**Beispiel 2**

100 Teile Polyether 3 und 3,9 Teile Polyisocyanat 4, sowie 1 Teil Dibutylzinndilaurat werden innerhalb von einer Minute intensiv vermischt und auf Siliconpapier zu einer 0,5 mm starken Schicht ausgegossen. Nach 10 Minuten beginnt die Gelbildungsreaktion unter langsamer Viskositätserhöhung der Reaktionsmischung. Es wird ein textiles Faservlies auf Basis von Viskose faltenfrei auf den hochviskosen Film der Reaktionsmischung aufgelegt.

Nach 20 Minuten ist das Gemisch zum Gel erstarrt. Das gelbeschichtete textile Faservlies wird in Stacke von jeweils 8 x 10 cm Größe zerteilt und auf der gelbeschichteten Seite mit jeweils 4 x 6 cm großen Wundmull-Stücken in der Weise versehen, daß rundum am Rand eine jeweils 2 cm breite haftfähige Gelschicht erhalten bleibt.

Der erhaltene Wundverband ist für die Versorgung von trockenen und leicht sezernierenden Wunden geeignet.

**Beispiel 3**

100 Teile Polyether 4 und 9,8 Teile Polyisocyanat 4 sowie 2 Teile Dibutylzinndilaurat werden innerhalb von einer Minute intensiv vermischt und auf einer Teflonplatte zu einer 0,1 mm starken Schicht ausgezogen. Nach 10 Minuten beginnt die Gelbildungsreaktion unter langsamer Viskositätserhöhung der Reaktionsmischung. Es wird ein elastisches Gewirke auf Basis von Polyamid/Polyurethan-Fasern faltenfrei auf den hochviskosen Film der Reaktionsmischung aufgelegt. Nach 20 Minuten ist sie zum Gel erstarrt. Der gelbeschichtete Stretchstoff wird in Streifen von 8 cm Breite zerteilt. Auf die gelbeschichtete Seite der Streifen werden jeweils 2,6 cm breite Streifen aus textilem Viskose-Vliesstoff (mit Gewebestruktur) in der Weise in Längsrichtung Zentral aufgeklebt, daß in Längsrichtung an den Rändern ein jeweils 2,7 cm breiter Bereich von haftfähigem Gel erhalten bleibt.

Die erhaltenen Streifen können in Querrichtung in Stücke von 1 bis 3 cm Breite zerteilt werden, die dann als Wundschnellverbände für die Versorgung von kleinen Wunden verwendet werden können.

**Beispiel 4**

100 Teile Polyether 2 und 5,3 Teile Polyisocyanat 4 sowie 1 Teil Dibutylzinnlaurat werden innerhalb von einer Minute intensiv vermischt und mit Hilfe einer Spritzpistole auf eine elastische Binde (Baumwoll-Schußfäden, gekräuselte Polyamid-Kettfäden) aufgesprüht, wobei die Auftragsmenge an Gelmasse 35 Teile pro Quadratmeter beträgt. Die Verfestigung zum Gel erfolgt nach 10 Minuten.

Die erhaltene gelbesprühte Binde ist zum Anlegen von Verbänden an konischen Körperpartien geeignet, da die einzelnen Bindenlagen sehr gut auf sich selbst haften und somit bei Bewegungen nicht verrutschen.

**Beispiel 5**

Ein Gemisch aus 100 Teilen Polyether 1, 3,6 Teilen Polyisocyanat 5 und 3 Teilen Dibutylzinndilaurat wird nach der in Beispiel 1 beschriebenen Verfahrensweise zur Beschichtung von einem elastischen Gewirke auf Basis von Polyamid (84 %) und Polyurethan (16 %) verwendet.

Die durch Zerteilen des beschichteten Stretchgewirkes erhaltenen Streifen von 3 cm Breite sind zur Fixierung von Kathetern und Sonden sowie von Notverbänden aus Wundmull geeignet.

**Beispiel 6**

Man verfährt genau so wie in Beispiel 5, verwendet jedoch anstelle der Kombination von Polyether 1 und Polyisocyanat 5 die nachfolgend aufgelisteten Kombinationen von Polyethern und Polyisocyanaten:
100 Teile werden abgemischt mit folgenden Ge
Polyether wichtsteilen an Polyisocyanaten

| 100 Teile Polyether | werden abgemischt mit folgenden Gewichtsteilen an Polyisocyanaten | | |
|---|---|---|---|
| Nr. 2 | 4,3 Teile | Polyisocyanat | 5 |
| Nr. 4 | 6,6 " | " | 5 |
| Nr. 5 | 8,5 " | " | 5 |
| Nr. 6 | 7,6 " | " | 5 |
| Nr. 1 | 3,6 " | " | 2 |
| Nr. 2 | 4,1 " | " | 2 |
| Nr. 3 | 2,46 " | " | 2 |
| Nr. 4 | 5,2 " | " | 2 |
| Nr. 7 | 5,0 " | " | 2 |
| Nr. 1 | 2,9 " | " | 1 |
| Nr. 3 | 1,8 " | " | 1 |
| Nr. 9 | 4,15 " | " | 2 |

7

**Beispiel 7**

Ein Gemisch aus 100 Teilen Polyether 7, 7,56 Teilen Polyisocyanat 4 und 2 Teilen Dibutylzinndilaurat wird innerhalb einer Minute intensiv vermischt und auf einer Teflonplatte zu einem 0,3 mm starken Film ausgezogen. Ein textiler Vliesstoff auf Viskose-Basis wird auf den durch die Reaktion hochviskos werdenden Film faltenfrei gelegt. Nach der beendeten Gelbildung wird der mit einem Gelfilm versehene Vliesstoff in 2 cm breite Streifen zerteilt.

Derartige Streifen eignen sich zur Befestigung von Informationsblättern oder Werbeplakaten auf Schaufensterscheiben.

**Beispiel 8**

Man verfährt genauso wie in Beispiel 7, verwendet jedoch anstelle der Kombination von Polyether 7 und Polyisocyanat 4 die nachfolgend aufgeführten Kombinationen von Polyethern und Polyisocyanaten:

| 100 Teile Polyether | werden mit folgenden Teilen an Polyisocyanat abgemischt |
|---|---|
| Nr. 8 | 9,9 Teile Polyisocyanat 4 |
| Nr. 1 | 3,14 " " 3 |
| Nr. 4 | 5,15 " " 3 |
| Nr. 10 | 8,8 " " 4 |

**Beispiel 9**

Es werden 100 Teile Polyether 2, 15 Teile eines Öls auf Basis Polydimethylsiloxan (Baysilone-Öl M 5000, Bayer AG) mit einer Viskosität von 5000 mm$^2$/s bei 25°C, 5 Teile Polyisocyanat 4 und 0,1 Teile Dibutylzinndilaurat miteinander intensiv vermischt, auf eine Teflonplatte gegossen und zu einer 0,1 mm starken Schicht ausgezogen.

Nach 10 Minuten ist die Viskosität sehr stark angestiegen. Es wird ein dehnbares Gewirke auf Basis von Polyamid/Polyurethan auf das hochviskose Reaktionsgemisch gelegt. Das gelbeschichtete Gewirke wird nach 30 Minuten von der Platte abgezogen und in Streifen von 3 cm Breite zerschnitten. Derartige Streifen können als Fixierstreifen im medizinischen Bereich, wie z. B. für die Befestigung von Verbänden, verwendet werden.

**Beispiel 10**

Es wird genauso verfahren wie in Beispiel 9, aber anstelle des Öls auf Basis Polydimethylsiloxan wird ein Öl auf Basis von polymerem Methylphenylsiloxan (Baysilone-Öl PH 300, Bayer AG) mit einer Viskosität von 300 mm$^2$/s bei 25°C eingesetzt.

Die erhaltenen gelbeschichteten Streifen können ebenfalls als Fixierstreifen im medizinischen Bereich verwendet werden.

Testmethode für Haftfestigkeit

Ein Gel-Streifen mit den Abmessungen 125 mm x 25 mm wird mit einer Kraft von 7,5 Newton auf eine saubere, glatte VA-Stahlplatte gepreßt und dann mit einer Geschwindigkeit von 100 mm/min wieder abgezogen. Mit einem x-y-Rekorder wird die Kraft, die zum Trennen von Probe und Substrat aufzuwenden ist, über den Trennweg aufgezeichnet. Aus dem erhaltenen Kraft-Weg-Diagramm wird die mittlere Kraft $\bar{F}$ über 75 mm Trennweg ermittelt. $\bar{F}$ sollte bei den erfindungsgemäßen Gelen mindestens 0,5 N, vorzugsweise mindestens 1 N, besonders bevorzugt mehr als 2 N betragen.

**Patentansprüche**

1. Selbsthaftende Flächengebilde, enthaltend mindestens eine Trägerschicht und mindestens eine Haftschicht, wobei mindestens eine der beiden Oberflächen des Flächengebildes mindestens teilweise mit einer Haftschicht bedeckt ist, dadurch gekennzeichnet, daß diese Haftschicht ein Gel ist, welches

(A) 15-62 Gew.-%, bezogen auf die Summe aus (A) und (B), eines kovalent vernetzten Polyurethans als hochmolekulare Matrix und

(B) 85-35 Gew.-%, bezogen auf die Summe aus (A) und (B), einer oder mehrerer in der Matrix durch Nebenvalenzkräfte fest gebundener Polyhydroxylverbindungen mit einem mittleren Molekulargewicht zwischen 1000 und 12 000 und einer mittleren OH-Zahl zwischen 20 und 112 als flüssigem Dispersionsmittel, wobei das Dispersionsmittel im wesentlichen frei ist an Hydroxylverbindungen mit einem Molekulargewicht unter 800, sowie

(C) 0-100 Gew.-%, bezogen auf die Summe aus (A) und (B), an Füll- und/oder Zusatzstoffen

enthält, und welches erhältlich ist durch Umsetzung einer Mischung von

a) einem oder mehreren Polyisocyanaten,

b) einer oder mehreren Polyhydroxylverbindungen mit einem mittleren Molekulargewicht zwischen 1000 und 12 000, und einer mittleren OH-Zahl zwischen 20 und 112,

c) gegebenenfalls Katalysatoren für die Reaktion zwischen Isocyanat- und Hydroxylgruppen sowie gegebenenfalls

d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxyverbindung (Fp) zwischen 3 und 6 beträgt und die Isocyanatkennzahl (K) der Formel

$$K = \frac{300 \pm X}{(F_I \cdot F_p) - 1} + 7$$

gehorcht, in welcher $X \leqslant 120$, vorzugsweise $X \leqslant 100$, besonders bevorzugt $X \leqslant 90$ ist und die Kennzahl K bei Werten zwischen 15 und 70 liegt.

2. Flächengebilde nach Anspruch 1, dadurch gekennzeichnet, daß das Gel

(A) 20-57 Gew.-%, bezogen auf die Summe aus (A) und (B), des kovalent vernetzten Polyurethans und

(B) 80-43 Gew.-%, bezogen auf die Summe aus (A) und (B), der Polyhydroxylverbindungen des flüssigen Dispersionsmittels

enthält.

3. Flächengebilde nach Anspruch 2, dadurch gekennzeichnet, daß das Gel

(A) 25-47 Gew.-% des kovalent vernetzten Polyurethans und

(B) 75-53 Gew.-% der Polyhydroxylverbindungen des flüssigen Dispersionsmittels

enthält.

4. Flächengebilde nach Anspruch 1 und 3, dadurch gekennzeichnet, daß die Polyhydroxylverbindungen des flüssigen Dispersionsmittels ein mittleres Molekulargewicht zwischen 1500 und 8000, insbesondere zwischen 2000 und 6000, aufweisen.

5. Flächengebilde nach Anspruch 1 bis 4, dadurch gekennzeichnet, daß die Polyhydroxylverbindungen des flüssigen Dispersionsmittels eine mittlere OH-Zahl zwischen 25 und 84, insbesondere zwischen 28 und 56, aufweisen.

6. Flächengebilde nach Anspruch 1 bis 5, dadurch gekennzeichnet, daß das flüssige Dispersionsmittel im wesentlichen frei ist an Hydroxylverbindungen mit einem Molekulargewicht unter 1000, insbesondere unter 1500.

7. Flächengebilde nach Anspruch 1 bis 6, dadurch gekennzeichnet, daß die Isocyanatkennzahl (K) zwischen 18 und 55, insbesondere zwischen 20 und 45, liegt.

8. Flächengebilde nach Anspruch 1 bis 7 in der Form eine medizinischen Pflasters, Wundverbandes, medizinischen Fixierstreifens oder einer rutschfesten starren oder elastischen Binde.

9. Verfahren zur Herstellung von Flächengebilden nach Anspruch 1 bis 8, dadurch gekennzeichnet, daß man ein Reaktionsgemisch aus

a) einem oder mehreren Polyisocyanaten,

b) einer oder mehreren Polyhydroxylverbindungen mit einem mittleren Molekulargewicht zwischen 1000 und 12 000, und einer mittleren OH-Zahl zwischen 20 und 112,

c) gegebenenfalls Katalysatoren für die Reaktion zwischen Isocyanat- und Hydroxylgruppen sowie gegebenenfalls

d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindungen ($F_p$) zwischen 3 und 6 beträgt und die Isocyanatkennzahl (K) der Formel

$$K = \frac{300 \pm X}{(F_I \cdot F_p) - 1} + 7$$

gehorcht, in welcher $X \leqslant 120$, vorzugsweise $X \leqslant 100$, besonders bevorzugt $X \leqslant 90$ ist, und die Kennzahl K bei Werten zwischen 15 und 70, bevorzugt zwischen 18 und 55, besonders bevorzugt zwischen 20 und 45 liegt, zu einem Gel umsetzt und während oder nach der Beendigung der gelbildenden Reaktion nach dem Direktoder Umkehrverfahren auf mindestens einen Teil einer Oberfläche eines Trägermaterials aufbringt.

10. Verwendung von Flächengebilden nach Anspruch 1 bis 7 als medizinische Pflaster, Verbandmaterialien, Fixierstreifen, Reparaturmaterialien, Haftetiketten oder Haftflächen für Insekten.

## Claims

1. Self-adhesive sheet-like structures containing at least one support layer and at least one adhesive layer at least one of the two surfaces of the sheet-like structure being at least partially covered with an adhesive layer, characterised in that this adhesive layer is a gel which contains

(A) 15-62 % by weight, relative to the total of and (B), of a covalently crosslinked polyurethane as the high molecular weight matrix and

(B) 85-38 % by weight, relative to the total of (A) and (B) of one or more polyhydroxy compounds which are firmly fixed in the matrix by secondary valence forces and have an average molecular weight between 1,000 and 12,000, and an average OH number between 20 and 112, as a liquid dispersing agent, the dispersing agent being essentially free of hydroxy compounds having a molecular weight below 800, and,

(C) 0-100 % by weight relative to the total of (A) and (B), of fillers and/or additives,

and which can be obtained by reaction of a mixture of

a) one or more polyisocyanates,

b) one or more polyhydroxy compounds having an average molecular weight between 1,000 and 12,000 and an average OH number between 20 and 112,

c) where appropriate, catalysts for the reaction between isocyanate and hydroxyl groups and, where appropriate,

d) fillers and additives which are known per se from polyurethane chemistry,

this mixture being essentially free of hydroxy compounds having a molecular weight below 800, the average functionality of the polyisocyanates $(F_I)$ being between 2 and 4, the average functionality of the polyhydroxy compound $(F_p)$ being between 3 and 6, and the isocyanate number (K) obeying the formula

$$K = \frac{300 \pm X}{(F_I \cdot F_p) - 1} \times 7$$

in which

$X \leqslant 120$, preferably $X \leqslant 100$, and particularly preferably $X \leqslant 90$ and the number K is between 15 and 70.

2. Sheet-like structures according to Claim 1, characterised in that the gel contains

(A) 20 - 57 % by weight, relative to the total of (A) and (B), of the covalently crosslinked polyurethane and

(B) 80 - 43 % by weight, relative to the total of (A) and (B), of the polyhydroxy compounds of the liquid dispersing agent.

3. Sheet-like structures according to Claim 2, characterised in that the gel contains

(A) 25 - 47 % by weight of the covalently crosslinked polyurethane and

(B) 75 - 53 % by weight of the polyhydroxy compounds of the liquid dispersing agent.

4. Sheet-like structures according to Claim 1 to 3, characterised in that the polyhydroxy compounds of the liquid dispersing agent have an average molecular weight between 1,500 and 8,000, in particular between 2,000 and 6,000.

5. Sheet-like structures according to Claim 1 to 4, characterised in that the polyhydroxy compounds of the liquid dispersing agent have an average OH number between 25 and 84, in particular between 28 and 56.

6. Sheet-like structures according to Claim 1 to 5, characterised in that the liquid dispersing agent is essentially free of hydroxy compounds having a molecular weight below 1,000, in particular below 1,500.

7. Sheet-like structures according to Claim 1 to 6, characterised in that the isocyanate number (K) is between 18 and 55, and in particular between 20 and 45.

8. Sheet-like structures according to Claim 1 to 7 in the form of a medical plaster, wound dressing, medical fixing tape or non-slip inelastic or elastic bandage.

10

9. Process for the production of sheet-like structures according to Claim 1 to 8, characterised in that a reaction mixture composed of

a) one or more polyisocyanates,

b) one or more polyhydroxy compounds having an average molecular weight between 1,000 and 12,000, and an average OH number between 20 and 112,

c) where appropriate, catalysts for the reaction between isocyanate and hydroxyl groups and, where appropriate,

d) fillers and additives known per se from polyurethane chemistry,

this mixture being essentially free of hydroxy compounds having a molecular weight below 800, the average functionality of the polyisocyanates ($F_I$) being between 2 and 4, the average functionality of the polyhydroxy compounds ($F_p$) being between 3 and 6, and the isocyanate number (K) obeying the formula

$$K = \frac{300 \pm X}{(F_I \cdot F_p) - 1} + 7$$

in which

$X \leqslant 120$, preferably $X \leqslant 100$, particular preferably $X \leqslant 90$, and the number K is between 15 and 70, preferably between 18 and 55, and particularly preferably between 20 and 45, is reacted to form a gel and is, during or after completion of the gel-forming reaction, applied by the direct or reverse process to at least one part of one surface of a support material.

10. Use of sheet-like structures according to Claim 1 to 7 as medical plasters, dressing materials, fixing tapes, repair materials, adhesive labels or adhesive surfaces for insects.

**Revendications**

1. Articles planiformes auto-adhérents, contenant au moins une couche de support et au moins une couche adhésive, l'une au moins des deux faces de l'article planiforme étant recouverte, en partie au moins d'une couche adhésive, caractérisés en ce que cette couche adhésive est un gel qui contient

A) 15 à 62 % en poids, par rapport à la somme de A et B, d'un polyuréthanne réticulé par covalence, en tant que gangue à haut poids moléculaire et

B) 85 à 38 % en poids, par rapport à la somme de A et B, d'un ou plusieurs composés polyhydroxylés fixés solidement dans la gangue par des forces de valences secondaires, à un poids moléculaire moyen de 1000 à 12 000 et un indice d'OH moyen de 20 à 112, en tant que milieu de dispersion liquide, le milieu de dispersion étant essentiellement exempt de composés hydroxylés de poids moléculaire inférieur à 800, et

C) 0 à 100 % en poids, par rapport à la somme de A et B, de matière de charge et/ou d'additifs,

et qui peut être obtenu par réaction d'un mélange de

a) un ou plusieurs polyisocyanates,

b) un ou plusieurs composés polyhydroxylés de poids moléculaire moyen 1000 à 12 000, indice d'OH moyen 20 à 112,

c) le cas échéant des catalyseurs pour la réaction entre les groupes isocyanates et les groupes hydroxy et le cas échéant

d) des matières de charge et additifs connus en soi dans la chimie des polyuréthannes,

ce mélange étant essentiellement exempt de composés hydroxylés de poids moléculaire inférieur à 800, la fonctionnalité moyenne des polyisocyanates $F_I$ étant de 2 à 4, la fonctionnalité moyenne du composé polyhydroxylé $F_P$ étant de 3 à 6 et la caractéristique d'isocyanate K satisfaisant à la formule

$$K = \frac{300 + X}{(F_I \cdot F_p) - 1} + 7$$

dans laquelle X est inférieur ou égal à 120, de préférence inférieur ou égal à 100, plus spécialement inférieur ou égal à 90, et la caractéristique K allant de 15 à 70.

2. Articles planiformes selon la revendication 1, caractérisés en ce que le gel contient

A) 20 à 57 % en poids, par rapport à la somme de A et B, du polyuréthanne réticulant par covalence et

B) 80 à 43 % en poids, par rapport à la somme de A et B, des composés polyhydroxylés du milieu de dispersion liquide.

3. Articles planiformes selon la revendication 2, caractérisés en ce que le gel contient
A) 25 à 47 % en poids du polyuréthanne réticulant par covalence et
B) 75 à 53 % en poids des composés polyhydroxylés du milieu de dispersion liquide.

4. Articles planiformes selon les revendications et 3, caractérisés en ce que les composés polyhydroxylés du milieu de dispersion liquide ont un poids moléculaire moyen de 1 500 à 8 000, plus spécialement de 2 000 à 6 000.

5. Articles planiformes selon les revendications 1 à 4, caractérisés en ce que les composés polyhydroxylés du milieu de dispersion liquide ont un indice d'OH moyen de 25 à 84, plus spécialement de 28 à 56.

6. Articles planiformes selon les revendications 1 à 5, caractérisés en ce que le milieu de dispersion liquide est essentiellement exempt de composés hydroxylés de poids moléculaire inférieur à 1 000, plus spécialement inférieur à 1 500.

7. Articles planiformes selon les revendications 1 à 6, caractérisés en ce que la caractéristique d'isocyanate K se situe entre 18 et 55, plus spécialement entre 20 et 45.

8. Articles planiformes selon les revendications 1 à 7, sous la forme d'emplâtres médicinaux, de pansements pour blessures, de bandes de fixation médicinales ou de bandages rigides ou élastiques résistant au glissement.

9. Procédé de préparation des articles planiformes selon les revendications 1 à 8, caractérisé en ce que l'on convertit en un gel un melange de reaction consistant en
a) un ou plusieurs polyisocyanates,
b) un ou plusieurs composés polyhydroxylés de poids moléculaire moyen 1000 à 12 000 et d'indice d'OH moyen 20 à 112,
c) le cas échéant des catalyseurs pour la réaction entre les groupes isocyanates et les groupes hydroxy et le cas échéant
d) des matières de charge et additifs connus en soi dans la chimie des polyuréthannes,
ce mélange étant essentiellement exempt de composés hydroxylés de poids moléculaire inférieur à 800, la fonctionnalité moyenne des polyisocyanates $F_I$ allant de 2 à 4, la fonctionnalité moyenne des composés polyhydroxylés $F_P$ allant de 3 à 6 et la caractéristique d'isocyanate K satisfaisant à la formule

$$K = \frac{300 \pm x}{(F_I \cdot F_P) - 1} + 7$$

dans laquelle X est inférieur ou égal à 120, de préférence inférieur ou égal à 100, plus spécialement inférieur ou égal à 90, la caractéristique K se situant entre 15 et 70, de préférence entre 18 et 55, plus spécialement entre 20 et 45,
et, durant ou après la réaction de formation du gel, on applique par le procédé direct ou par le procédé avec inversion sur une partie au moins d'une face d'une matière de support.

10. Utilisation des articles planiformes selon les revendications 1 à 7 en tant qu'emplâtres médicinaux, matériaux de pansements, bandes de fixation, matériaux de réparation, étiquettes collantes ou surfaces collantes pour insectes.